# EUROPEAN PATENT APPLICATION

(11) **EP 3 636 140 A1**
(43) Date of publication of application: **15.04.2020**
(21) Application number: 18199524.2
(22) Date of filing: 10.10.2018
(51) Int. Cl.: A61B 5/00

(54) **SYSTEM AND METHOD FOR SENSOR POSITIONING AND PLACEMENT FOR A SENSOR ARRANGEMENT**

(71) Applicant: Koninklijke Philips N.V., 5656 AG Eindhoven (NL)
(72) Inventor: SISODIA, Rajendra Singh, 5656 AE Eindhoven (NL); CHAUDHURY, Sudipta, 5656 AE Eindhoven (NL); BHAT, Ravindra, 5656 AE Eindhoven (NL); VUPPALA, Sunil Kumar, 5656 AE Eindhoven (NL); JOHNSON, Mark Thomas, 5656 AE Eindhoven (NL); VOGTMEIER, Gereon, 5656 AE Eindhoven (NL); DJAJADININGRAT, Johan Partomo, 5656 AE Eindhoven (NL)
(74) Representative: de Haan, Poul Erik

(57) **Abstract**

The invention may improve measurement quality of sensor by placing them at the optimal position. The invention is directed to a sensor positioning system (1) for positioning and placement of a sensor arrangement on a patient's body. The sensor positioning system (1) comprises a sensor arrangement (10) comprising a first sensor (11) and a second sensor (12), a placement tracker (20), and a processor (30). The placement tracker (20) is configured to track a position and/or orientation of the second sensor (12) relative to the first sensor (11) to determine an actual position and/or orientation of the second sensor (12) on the patient's body (2). The processor (30) is configured to compare the actual position and/or orientation of the second sensor (12) with a desired position and/or orientation of the second sensor (12) and to generate an output signal (35) indicating a difference between the actual position and/or orientation of the second sensor (12) and the desired position and/or orientation of the second sensor (12).

## Description

### FIELD OF THE INVENTION

The present invention relates generally to sensor placement and positioning, in particular to a sensor positioning system for positioning and placement of a second sensor relative to a first sensor, an examination arrangement, a method for positioning and placement of a sensor arrangement on a patient's body, a program element and a non-transitory computer-readable medium.

### BACKGROUND OF THE INVENTION

Sensors play a big role during imaging by providing vital physiological condition of patients as well as timings during scan. The sensors can be of different types such as Electrocardiography (ECG), respiratory rate (RR), Photoplethysmogram (PPG), saturation of peripheral oxygen (SPo2), or even patches (temperature, skin conductance, perspiration). Input from sensor can be used to auto-trigger a scan or specific part of scan such as cardiac cycle or reconstruction to compensate for motion. Further, ECG/RR may be used as input for scan gating. Sensor placement and the position of a sensor during a scan of an imaging modality is essential but a complex process. If such sensors are not placed properly or are positioned wrongly, the scan may have artefacts or even need to be repeated. Sensor placement on a patient's body may be a subjective process. They are based on physical palpation or using anatomical markers. Some sensor, such as SPo2 can be used by anybody, whereas sensor such as "ECG with multi-channel" needs to be placed by experienced staff. Some sensors, such as ultrasonic sensors not only need to be placed properly (no air gap) but also positioned properly (to isolate a vascular, tissue, anatomical region with right angle, depth etc.).

The scan from big modality requires multiple types of sensors, also referred as sensor arrangement. Such a sensor arrangement, which may comprise a plurality of sensors and different types of sensors makes it even more complex for placement and position to ensure that not only they are placed properly relative to themselves but also with respect to each other. For example, ECG leads should be placed properly with respect and relative to each other and other sensors, such as ultrasonic, should be placed in such a way that they do not introduce interferences.

### SUMMARY OF THE INVENTION

There may therefore be a need to improve the placement and the positioning of a sensor arrangement on the patient's body.

The object of the present invention is solved by the subject-matter of the independent claims, wherein further embodiments are incorporated in the dependent claims. According to a first aspect, a sensor positioning system for positioning and placement of a sensor arrangement on a patient's body is provided. The sensor positioning system comprises a sensor arrangement comprising a first sensor and a second sensor, a placement tracker and a processor. The placement tracker is configured to track and provide continuous feedback about a position and/or orientation of the second sensor relative to the first sensor to determine an actual position and/or orientation of the second sensor on the patient's body. The processor is configured to compare the actual position and/or orientation of the second sensor with a desired position and/or orientation of the second sensor and to generate an output signal indicating a difference between the actual position and/or orientation of the second sensor and the desired position and/or orientation of the second sensor.

Thus, the sensor positioning system may be used for positioning and placement (position and orientation) of a second sensor in view of a first sensor. The sensor arrangement may comprises at least two sensors, two sets of sensors or two types of sensors. The first sensor or the first set may be placed at a reference position on the patient's body, e.g. the sternum. It should be noted that the first sensor might be placed at potentially a single reference point. Alternatively or in addition, the first set of sensors may be placed at two reference points or even a triangulation of three reference points. Further, the first sensor may be a dummy sensor, which purpose solely it is to form a reference point for the second sensor, wherein the first sensor is placed at an easy to locate reference position.

It should be noted that the term position and/or orientation includes the position in coordinates and the orientation with respect to the coordinate system. As coordinate system, Cartesian coordinates (X;Y;Z) may be used and the orientation may be determined based on the rotation around the coordinate axes.

The relative position and/or orientation of the second sensor in view to the first sensor may be tracked by a placement tracker.

The sensor arrangement may be associated with a position profile, for position and placement of its sensors as per profile. This profile may be transmitted to or stored on the processor of the sensor positioning system. The position profile may be the desired placement grid for different sensor types over a curvature surface, e.g. the patient's body, wherein the placement grid points may be 3D coordinate points, with or without a specified orientation of the sensor. It should be noted that a position profile may be scan dependent, modality dependent, patient dependent or based on empirical values. The position profile may comprise a reference position, wherein the reference position is associated with one reference sensor, i.e. the first sensor of the sensor arrangement, and the desired position for the second sensor is defined with respect to the first and reference sensor, respectively. The first sensor may anchored using an anatomical marker on the patient's body or via an anatomical feature detection from the first sensor.

Thus, the actual position of the second senor may be determined based on the reference position of the first sensor and the relative position of the second sensor relative to the first sensor. The tracking of the relative position and/or orientation of the second sensor in view of the first sensor may be continuously performed using the placement tracker. The placement tracker may be realized by an optical shape sensor or via a body channel communication sensor.

The processor may compare the actual position and/or orientation of the second sensor with the desired position and/or orientation of the second sensor. Further, the processor may generate an output signal based on this comparison. This output signal may indicate the difference between the actual position and/or orientation and the desired position. Thus, the output signal may be a feedback signal to the user of the sensor positioning system.

It should be noted that the processor might be an electrical circuit, a control unit or a calculation device, which also comprises a memory device to store data. Further, the processor may also contain a transceiver system to receive the position profile or the desired positions and/or orientations or connect to an external system, which may act as feedback system.

Further, the placement tracker itself may provide feedback for each placement via a feedback system, the feedback system is preferably a multi-color fibre optic or an RF transceiver device operating at body channel communication frequencies of around 100MHz.

It should be noted that the second sensor might be positioned and placed by self-guidance where the tracking coordinates are dynamically evaluated with respect to the placement profile and are guided considering the current coordinate and placement profile. Color changing optical fibre can be used to dynamically provide feedback and guidance on the placement. Therefore, the use of the sensor positioning system ensures an easy and reliable positioning and placement of the sensor arrangement on a patient's body. Further, the sensor arrangement may be positions faster and also by untrained staff during the scan procedure to reduce time and improve the performance.

In an autonomous or self-guided scan procedure, e.g. to minimize the role of support staff for patient preparation during the scan, it may be required to have a self-guided mechanism that not only allows diverse sensors to be placed but also actively guide them for placements and inform about change of placement. Further, personalization of sensor placement on the patient's body for a scan may be needed to avoid errors during preparation.

According to an embodiment, the placement tracker is an optical shape sensor. Dynamic fiber-optic shape sensing, often also referred to as curvature or bend sensing or as Fibre Optical Shape Sensing (FOSS), is demonstrated using fiber segment interferometry, where chains of fiber segments, separated by broadband bragg grating reflectors, are interrogated using range-resolved interferometry.

The placement tracker may be based on dual-layer fiber bragg grating arrays with orthogonal mesh structure, which enable 3D bi-directional shape sensing.

Further, the optical shape sensor may be configured to track and to provide continuous feedback about the position of a sensor inside a body, e.g. tip of a needle in a catheter using shape sensing. Furthermore, 3D shape sensing is proposed to detect surface curvature, e.g. to detect strain.

Alternatively or in addition, the proposed sensor positioning system uses a 3D shape sensing technique to provide a self-referencing position and placement guidance for the sensor arrangement. Further, measuring data and feedback information, such as colors and light patterns may be transmitted through the optical shape sensor. In particular, the shape of the optical shape sensor may be determined by the change color of photons in the fibre optics cable.

Furthermore, since an optical link exist between the first and the second sensor sensors, they can also communicate over this optical link about the position and/or orientation and transmit measurement data to the processor for further processing.

According to another embodiment, the placement tracker is a RF transceiver device operating at a body channel communication frequency, in particular using frequencies around 100MHz.

A further approach for positioning of sensors on the patient's body is the body channel positioning approach. Hereby the sensors may be provided with a pair of capacitive electrodes (e.g. two parallel electrodes) which capacitively contact the skin and can both transmit and receive signals propagated across and/or over the surface of the body. In some situations there are advantageously multiple capacitive electrode pairs on a given sensor (e.g. to assist positioning and orientation). Further, in this case the second sensor may be equipped with a guiding system such as a LED or a speaker to provide feedback over the actual position and/or orientation of the second sensor to the user.

According to an embodiment, the reference position and/or orientation is determined based on a deterministic algorithm, which is based on position, weightage, computational capabilities or sensing and power attributes.

Thus, to determine the reference position and/or orientation or reference point for the first sensor of the sensor arrangement, deterministic algorithms may be used to elect the master reference point(s) based on position/orientation, weightage, computational capabilities, sensing and power attributes. Weightage may be determined by various factors including the positioning data of earlier scans of same patient. The accuracy and stability of previous scans of the same/similar patient with respect to movement and quality of the scans influence the value of weightage. This addresses the need of personalization of sensor placement on the patient for scan to avoid errors during preparation. Further, may be N reference positions are determined to deal with sensor node failures. After the election of reference positions, all sensors will automatically determine their relative positions.

According to an embodiment, the processor is configured to determine whether the first sensor is accurately placed at a reference position and/or orientation on the patient's body by an iterative evaluation of the measurement quality of the first sensor.

Thus, the measurement data of the first sensor may be analyzed by the processor in view of measurement quality. If the measurement quality exceeds a predefined threshold, the first sensor is accurately placed at the reference position and/or orientation on the patient's body.

According to an embodiment, the sensor positioning system further comprises a receiver. The receiver is configured to receive the reference position and/or orientation for the first sensor, and the receiver is configured to receive the desired position and/or orientation for the second sensor. The receiver may receive the desired position and/or orientation and/or the reference position and/or orientation from a sending unit. This sending unit may be integrated into an examination apparatus, such as an MRI or CT. Further, the receive may receive the desired positions/orientations from a data base or by a calculation unit which determines the desired positions based on patient's data, such as weight, height, gender, age or an actual or previous body scan. The receiver may receive the desired positions via a wire or wireless over the air, e.g. over Wi-Fi or Bluetooth.

Alternatively or in addition, the desired position and/or orientation and/or the reference position and/or orientation of the sensors of the sensor arrangement may be stored on the sensor positioning system, e.g. on a memory unit. In particular, if the desired position and/or orientation or the reference position and/or orientation does not change for the sensors of the sensor arrangement or if the distances between the first and second sensor remain stable, the positions may be stored directly on the sensor positioning system.

According to an embodiment, the reference position and/or orientation is an anatomical marker of the patient's body, such as the sternum or the carotid artery of the patient. The orientation may be ensured based on labeling on the first sensor.

According to an embodiment, the desired position and/or orientation for the second sensor is determined based on an image of the patient's body, e.g. a X-ray, a MRI or a CT image, or based on empirical values.

According to an embodiment, the output signal of the processor is an optical and/or acoustical feedback. For example, the optical feedback may be displayed on a display and the acoustical feedback may be emitted by a speaker.

According to an embodiment, the processor is configured to provide a guidance for the placement of the second sensor at the desired position and/or orientation based on the comparison of the actual position and/or orientation of the second sensor with the desired position and/or orientation of the second sensor.

Thus, the user may be actively be guided to place the second sensor at the desired position and/or orientation on the patient's body. For example, the sensor positioning system may display that the second sensor should be positioned and placed further right or left, or should be rotated for 15°.

According to an embodiment, the optical feedback and/or the guidance for the placement of the second sensor comprises different colors and/or different light patterns. In other words, the guidance for the user may be performed by different colors or light patterns including spatial or temporal light pattern, i.e. animation. The guidance for the patient could be done via a simple color-coding, which is explained to the patient before. For example, colors as green, yellow, blue and purple could be used to indicate the movement directions (up, down, left, and right) for reaching the desired position and/or orientation - a red light could be used to stop movement and indicate "best position". If the optical fibres are used as placement tracker, the optical fiber itself could be used as optical interface to visualize the color. In case of the body channel network the sensor itself may be equipped with a multi-color LED as user interface.

For calibration and validation of the position and/or orientation of the sensor with respect to the exact body region, the positioning of the sensor in the CT/MRI image via a CT/MRI visible structure would help to validate the position and/or orientation information and allows for recalibration.

Flashing lights might indicate a problem with the sensor position and/or orientation that might have moved or in case of skin contact sensors, the interface condition, e.g. gel, might have changed. Thus, the optical feedback can be used to indicate the need for action. This may also be performed in a synchronized way with an examination arrangement, as no position and/or orientation change should be done during image acquisition but only before the next scan. Audio guidance might support the patient actions.

The sequence of sensor placement can also be indicated by the optical interface either by a unique color, e.g. white flashing indicating the sensor that has to be placed next, and/or temporal switching sequence.

In case of display availability in the preparation area and/or inside the examination apparatus when the sensors should be placed the synchronized visualization of sensor number and senor position and/or orientation on the body can be shown.

Further, the patient may place the sensor arrangement on his body himself. Assuming the patient is lying down for an MRI or CT scan, a ceiling mounted mirror could allow the patient to see himself and the sensor arrangement from above. In this case, the examination arrangement may have built-in lighting elements to guide the user. For example, if the right hand edge lights up green it means that the user needs to move in the direction of that edge. If it lights up orange it means that the user is almost there, if it lights up red the user needs to stop. If the user overshoots and needs to reverse motion then the left hand edge lights up.

According to an embodiment, the optical shape sensor is configured to track and provide continuous feedback about the position and/or orientation of the second sensor relative to the first sensor and to provide optical guidance for the placement of the second sensor via different colors and/or different light patterns simultaneously. Further, the optical guidance may be supported or assisted by acoustical guidance to further increase the usability of the sensor positioning system.

According to an embodiment, the sensor positioning system further comprises a plurality of second sensors. Thus, the sensor arrangement may comprise two, three, four or a plurality of sensors. These sensors may be the same type or different types of sensors. Further, the first sensor may be a dummy sensor, which is only used for positioning at the reference position, which may form the basis for the positioning of the second sensors as the positions of the second sensors are determined based on a relative measurement in view of the first sensor.

According to an embodiment, the first and/or the second sensor is an ECG sensor, a respiratory rate sensor, a PPG sensor, a SPo2 sensor, a temperature sensor, a skin conductance sensor, an ultrasonic sensor, an AED pad and/or perspiration sensor.

According to a further aspect, an examination arrangement is provided. The examination arrangement comprises an examination apparatus for examination of an object of interest and a sensor positioning system described before and hereinafter. The examination apparatus may be a MRI or CT apparatus. Further, the sensor positioning system and the examination apparatus may interact, such that the examining apparatus transmits the desired positions of the first and/or second sensors of the sensor arrangement to the sensor positioning system. Thus, an optimized measurement of vital data of the patient may be achieved. Further, the examination apparatus may trigger measurements of the sensor arrangement for or based on the measurement data of the sensor arrangement the examination apparatus may be triggered. For example, the respiratory cycle may be determined based on the measurement data und based on the respiratory cycle the examination apparatus may be controlled. Further, the examination apparatus may be configured to obtain image data of the patient's body to determine the desired positions for the sensor arrangement.

According to another aspect, there is provided a method for positioning and placement of a sensor arrangement on a patient's body, comprising the steps of:
- Tracking and providing continuous feedback about the position and/or orientation of a second sensor relative to a first sensor by a placement tracker to determine the actual position and/or orientation of the second sensor on the patient's body;
- Comparing the actual position and/or orientation of the second sensor with the desired position and/or orientation of the second sensor to generate an output signal indicating a difference between the actual position and/or orientation of the second sensor and the desired position and/or orientation of the second sensor.

According to an embodiment the method further comprises the step of:
- Receiving a reference position and/or orientation on the patient's body for the placing the first sensor;
- Receiving the desired position and/or orientation on the patient's body for placing the second sensor;
- Checking whether the actual position and/or orientation of the second sensor is at the desired position/orientation,
if yes:
- Issuing an optical feedback confirming that the second sensor is at the desired position and/or orientation on the patient's body; and
if no:
- Guiding a user, via the optical feedback, to place the second sensor at the desired position/orientation.

According to another aspect, there is provided a computer program element controlling the system as previously described which, if the computer program element is executed by the processor, is adapted to perform the method steps as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the accompanying drawings:
Figure 1 shows block diagram of a sensor positioning system according to an embodiment of the invention.
Figure 2 shows an optical shape sensor used in the sensor positioning system according to an embodiment of the invention.
Figure 3 shows a patient with a sensor positioning system according to an embodiment of the invention.
Figure 4 shows an examining table with a patient and the sensor positioning system according to an embodiment of the invention.
Figure 5 shows a flow chart of a method for positioning and placement of a sensor arrangement on a patient's body.

### DETAILED DESCRIPTION OF EMBODIMENTS

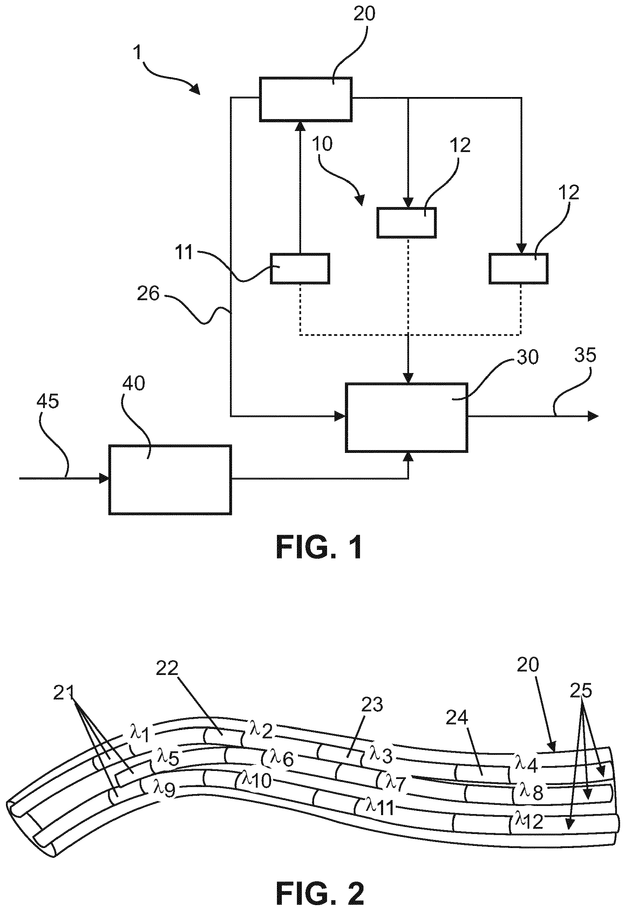

Fig. 1 shows a block diagram of a sensor positioning system 1. The sensor positioning system 1 comprises a sensor arrangement 10 with a first sensor 11 and at least one-second sensor 12, a placement tracker 20 and a processor 30. Further, the sensor positioning system 1 may comprise a receiver 40 to receive data 45, such as desired positions for the sensors 11, 12 of the sensor arrangement 10, i.e. sensor positioning profile. In Fig. 1 two second sensors 12 are shown, however, it should be understood that also a plurality of second sensors 12 might be used. The sensors 11, 12 of the sensor arrangement 10 are configured to be placed on a patient's body to measure vital data of the patient.

The sensors 11, 12 of the sensor arrangement 10 may are an ECG sensor, a respiratory rate sensor, a PPG sensor, a SPo2 sensor, a temperature sensor, a skin conductance sensor, an ultrasonic sensor an Automated external defibrillator (AED) pad and/or perspiration sensor. The placement tracker 20 is configured to track and provide continuous feedback about a position and/or orientation of the second sensor 12 relative to the first sensor 11. Thus, the placement tracker 20 is configured to determine an actual position and/or orientation of the second sensor 12 on the patient's body. The placement tracker 20 may be an optical shape sensor. This optical shape sensor may connect the second sensor 12 to the processor 30 and transmit at the same time measurement data from the second sensor 12 to the processor 30. Furthermore, the optical shape sensor may guide visible light, different colors or different light patterns to provide an optical feedback to the user of the sensor position system 1. In another embodiment, the placement tracker 20 may be a RF transceiver device operating at a body channel communication frequency. The body channel communication frequency may be around 100MHz. Thereby the sensors 11, 12 of the sensor arrangement 10 are provided with a pair of capacitive electrodes (e.g. 2 parallel electrodes), which capacitively contact the skin of the patient and can both transmit and receive signals propagated across the body. In some situations there are advantageously multiple capacitive electrode pairs on a sensor 11, 12 (e.g. to assist positioning and orientation).

It should be understood that the first sensor 11 should be accurately placed at a reference position and/or orientation on the patient's body. This reference position and/or orientation may remain the same for the first sensor 11 and be stored in the sensor positioning system 1 or be received via the receiver 40. In particular, the reference position and/or orientation may be an anatomical marker of the patient's body such as the sternum or the carotid artery of the patient. Further, the reference position and/or orientation for the first sensor 11 may be determined based on an image, such as a MRI, CT or X-ray image, of the patient's body, based on a previous body scan of the patient or based on empirical values. Further, the reference position and/or orientation may be determined based on a deterministic algorithm, which is based on position, weightage, computational capabilities or sensing and power attributes of the first sensor 11. Furthermore, the processor 30 is configured to determine whether the first sensor 11 is accurately placed at the reference position and/or orientation by an iterative evaluation of the measurement quality of the first sensor 11. In other words, if the measurement signal of the first sensor 11 is too low, the processor instruct to place the first sensor 11 at another position and/or orientation at which the measurement is repeated. This procedure is executed until the measurement quality exceeds a predefined threshold.

The desired position and/or orientation for the second sensor 12 may be stored in the sensor positioning system 1, e.g. 5cm apart from the first sensor, or may be received by the receiver 40. In addition, the desired position and/or orientation for the second sensor 12 may be determined based on image data, on a previous body scan of the patient or on empirical values. The received position and/or orientation data 45 for the sensors 11, 12 of the sensor arrangement 10 may be received via wire or wireless, e.g. via Bluetooth or Wi-Fi. These position and/or orientation data 45 may be stored in the receiver 40 and be transmitted to the processor 30. Further, also the measurement data from the sensors 11, 12 of the sensor arrangement 10 are transmitted to the processor (indicated by dotted lines) as well as the actual position and/or orientation 26 of the second sensors 12 tracked by the placement tracker 20.

The processor 30 compares the actual position and/or orientation of the second sensors 12 with their respective desired positions. Further, the processor 30 generates an output signal 35 indicating a difference between the actual position and/or orientation of the second sensors 12 and their respective desired positions.

The output signal 35 may be an optical feedback, such as a light, colors or light patterns. For example, the processor may instruct a LED to emit a red light or a flashing light as long the actual position and/or orientation of the second sensor 12 is not equal to the desired position and/or orientation and if the actual position and/or orientation of the second sensor 12 is equal to the desired/orientation position, the LED may emit a green light or a permanent light. Furthermore, the output signal 35 of the processor 30 may guide the user of the sensor position system 1 for positioning the second sensor 12 at the desired position. The guidance for the patient could be done via a simple color-coding that is explained to the patient before. For example, the colors green, yellow, blue and purple could be used to indicate the movement directions (up, down, left, and right) for reaching the desired position and/or orientation - a red light could be used to stop movement and indicate "best position". It should be noted, if the placement tracker 20 is the optical shape sensor, the fiber itself could be used as optical interface to visualize the color. In case of the body channel network the sensor itself has to be equipped with a multicolor LED as user interface.

Furthermore, flashing lights might indicate a problem with the sensor position and/or orientation that might have moved or in case of skin contact sensor the interface condition (e.g., gel) might have changed.

It should be understood that the optical feedback might be used to indicate the need for action by the user. Further, the optical feedback may be done in a synchronized way with an examination arrangement, since no position and/or orientation change should be done during image acquisition but only before the next scan. Furthermore, audio guidance may support the action of the user. Thus, the user may be the patient itself. In other words, due to the guiding ability of the sensor positioning system 1, the user does not have to be a professional trained medical assistance. Furthermore, the measurement quality of the sensor arrangement 10 may be improved as the positioning of the second sensors 12 are controlled and monitored. In addition, the placement of the sensor arrangement 10 may be accelerated as a direct feedback of an accurate placement is given to the user.

The sequence of the placement of the second sensors 12 may also be indicated by the optical interface, either by a unique color (white flashing indicating the sensor that has to be placed next) and/or temporal switching sequence. In case of display availability in the preparation area and/or inside the examination arrangement when the sensors should be placed the synchronized visualization of sensor number and senor position and/or orientation on the body may be displayed.

Fig. 2 shows an optical shape sensor used in the sensor positioning system as placement tracker 20 with a plurality of optical fibres 25. The placement tracker 20 comprises three different optical fibres 25. The optical shape sensor is also known as Fiber Optic Shape Sensing (FOSS). Hereby an optical fibre is used to determine the shape. Therefore, a plurality of fiber bragg gratings 21, 22, 23, 24 are introduced into the fibre optics 25 at predefined positions along the fibre optics 25. If the fibre optics 25 is bend, the wavelength changes accordingly, which is detected since the braggs have different indexes λ₁ - λ₁₂. Thus, the detected wavelength may conclude to the actual shape of the placement tracker 20. In the shown embodiment, each of the fibre optics 25 comprise four fiber bragg gratings 21, 22, 23, 24 with different indexes λ₁ - λ₅,λ₆ - λ₈ or λ₉ - λ₁₂. Further, since the sole fibre optics 25 are arranged offset to the middle of the placement tracker 20, each of the fibre optics 25 bends differently in the fiber bragg gratings 21, 22, 23, 24 and therefore, a 3D shape of the placement tracker 20 may be determined.

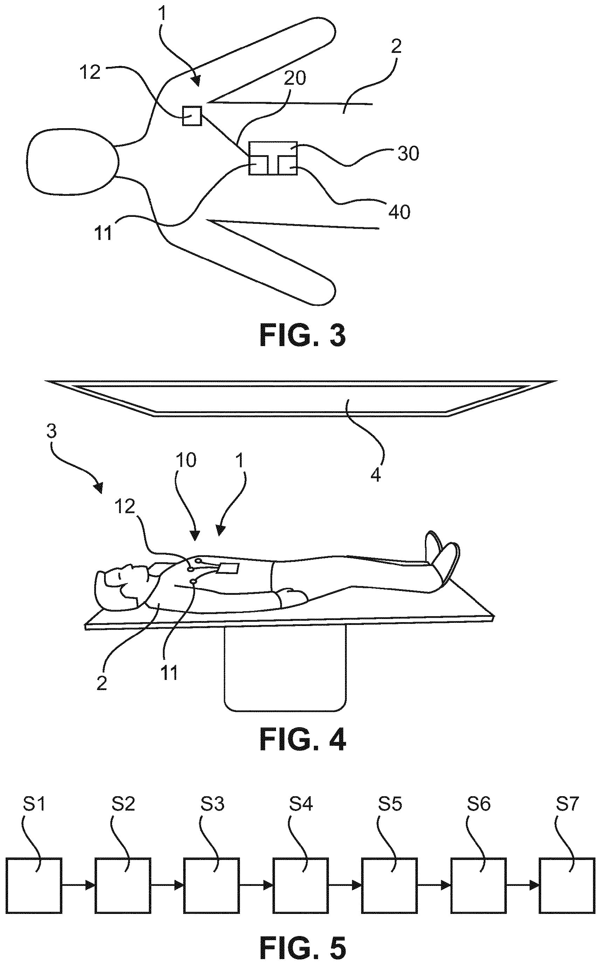

Fig. 3 shows a patient 2 with a sensor positioning system 1. In this embodiment the first sensor 11, the receiver 40 and the processor 30 are arranged in one common housing. Further, the placement tracker 20 is used to connect the first sensor 11 with the second sensor 12. During operation of the sensor positioning system 1, the first sensor 11 is placed at the reference position. Here the sternum of the patient 2. The desired position and/or orientation for the second sensor 12 is received by the receiver 40. Subsequently the second sensor 12 is moved over the patient's 2 body meanwhile the actual position and/or orientation of the second sensor 12 is tracked by the placement tracker 20. The processor 30 is configured to compare the actual position and/or orientation with the desired position and/or orientation of the second sensor 12. Further, the processor 30 generates the output signal in form of an optical and/or acoustical feedback, e.g. illuminating the second sensor, to guide the user to place the second sensor at the desired position.

In Fig. 4 an examining table 3, on which a patient 2 lies, is shown. The patient 2 is lying down for an MRI or CT scan, a ceiling mounted mirror 4 could allow the patient to see himself and the sensor arrangement 10 from above. If the left, top, right and bottom edges of the mirror may a built-in lighting element then those edges can be used to intuitively indicate in which direction the second sensor 12 has to be moved. For example if the right hand edge lights up green it means that the user needs to move the second sensor 12 in the direction of that edge. If it lights up orange it means that the user is almost there, if it lights up red the user needs to stop. If the user overshoots and needs to reverse motion then the left hand edge lights up.

Fig. 5 shows a flow chart for a method for positioning and placement of a sensor arrangement on a patient's body. In a first step S1, a reference position and/or orientation for placing a first sensor is received by a receiver. In step S2, the receiver receives a desired position and/or orientation of a second sensor. Subsequent, in step S3 a placement tracker tracks and provides continuous feedback about the position and/or orientation of the second sensor relative to the first sensor to determine the actual position and/or orientation of the second sensor on the patient's body. In step S4, a processor compares the actual position and/or orientation of the second sensor with the desired position and/or orientation of the second sensor and generates an output signal based on the difference between the actual position and/or orientation and the desired position and/or orientation of the second sensor. In step S5, it is checked whether the second sensor is at the desired position. If yes, step S6 is performed in which an optical feedback is issued to place the second sensor at the actual position. If step S5 is not fulfilled, the method performs step S7, in which a user is guided via the optical feedback to place the second senor at the desired position.

In another exemplary embodiment, a computer program or computer program element is provided that is characterized by being configured to execute the method steps of the method according to one of the preceding embodiments, on an appropriate system or device.

The computer program element might therefore be stored on a computer unit, which might also be part of an embodiment. This computing unit may be configured to perform or induce performing of the steps of the method described above. Moreover, it may be configured to operate the components of the above described device and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses invention.

Further, on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to the positioning of the sensor arrangement whereas other embodiments are described with reference to the method for positioning a plurality of sensors. However, a person skilled in the art will gather from the above and the following description that, unless otherwise notified, in addition to any combination of features belonging to one type of subject matter, also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 1: sensor positioning system
- 2: patient's body
- 3: examining table
- 4: mirror
- 10: sensor arrangement
- 11: first sensor of the sensor arrangement
- 12: second sensor of the sensor arrangement
- 20: placement tracker
- 21: first node of an optical shape sensor
- 22: second node of an optical shape sensor
- 23: third node of an optical shape sensor
- 24: fourth node of an optical shape sensor
- 25: shape sensing fibre optics
- 26: actual positions of the second sensor
- 30: processor
- 35: output signal of the processor
- 40: receiver
- 45: received position and/or orientation signals

## Claims

1. A sensor positioning system (1) for positioning and placement of a sensor arrangement on a patient's body, comprising:
- a sensor arrangement (10) comprising a first sensor (11) and a second sensor (12);
- a placement tracker (20); and
- a processor (30),
wherein the placement tracker (20) is configured to track and provide continuous feedback about a position and/or orientation of the second sensor (12) relative to the first sensor (11) to determine an actual position and/or orientation of the second sensor (12) on the patient's body (2), and
wherein the processor (30) is configured to compare the actual position and/or orientation of the second sensor (12) with a desired position and/or orientation of the second sensor (12) and to generate an output signal (35) indicating a difference between the actual position and/or orientation of the second sensor (12) and the desired position and/or orientation of the second sensor (12).

2. The sensor positioning system (1) according to claim 1,
wherein the placement tracker (20) is an optical shape sensor.

3. The sensor positioning system (1) according to claim 1,
wherein the placement tracker (20) is a RF transceiver device operating at a body channel communication frequency, in particular using frequencies around 100MHz.

4. The sensor positioning system (1) according to any one of the preceding claims,
wherein the processor (30) is configured to determine whether the first sensor (11) is accurately placed at a reference position and/or orientation on the patient's body by an iterative evaluation of the measurement quality of the first sensor (11).

5. The sensor positioning system (1) according to any one of the preceding claims, further comprising:
- a receiver (40),
wherein the receiver (40) is configured to receive the reference position and/or orientation for the first sensor (11), and
wherein the receiver (40) is configured to receive the desired position and/or orientation for the second sensor (12).

6. The sensor positioning system (1) according to any one of the preceding claims,
wherein the reference position and/or orientation is an anatomical marker of the patient's body (2), such as the sternum or the carotid artery of the patient.

7. The sensor positioning system (1) according to any one of the preceding claims,
wherein the desired position and/or orientation for the second sensor (12) is determined based on an image of the patient's body (2) or based on empirical values.

8. The sensor positioning system (1) according to any one of the preceding claims,
wherein the output signal (35) of the processor is an optical and/or acoustical feedback.

9. The sensor positioning system (1) according to any one of the preceding claims,
wherein the processor (30) is configured to provide a guidance for the placement of the second sensor (12) at the desired position and/or orientation based on the comparison of the actual position and/or orientation of the second sensor (12) with the desired position and/or orientation of the second sensor (12).

10. The sensor positioning system (1) according to claims 8 and 9,
wherein the optical feedback and/or the guidance for the placement of the second sensor (12) comprises different colors and/or different light patterns.

11. An examination arrangement, comprising:
- an examination apparatus for examination of an object of interest; and
- a sensor positioning system (1) according to any one of the claims 1 to 10.

12. A method for positioning and placement of a sensor arrangement on a patient's body, comprising the steps of:
- Tracking (S3) and providing continuous feedback about the position and/or orientation of a second sensor relative to a first sensor by a placement tracker to determine the actual position and/or orientation of the second sensor on the patient's body;
- Comparing (S4) the actual position and/or orientation of the second sensor with the desired position and/or orientation of the second sensor to generate an output signal indicating a difference between the actual position and/or orientation of the second sensor and the desired position and/or orientation of the second sensor.

13. The method according to claim 12, further comprising the step of:
- Receiving (S1) a reference position and/or orientation on the patient's body for the placing the first sensor;
- Receiving (S2) the desired position and/or orientation on the patient's body for placing the second sensor;
- Checking (S5) whether the actual position and/or orientation of the second sensor is at the desired position/orientation,
if yes:
- Issuing (S6) an optical feedback confirming that the second sensor is at the desired position and/or orientation on the patient's body; and
if no:
- Guiding (S7) a user, via the optical feedback, to place the second sensor at the desired position/orientation.

14. A computer program element for controlling a sensor positioning system according to any one of claims 1 to 10 or an examination arrangement of claim 11, which when being executed by a processor is configured to carry out the method according to any one of claims 12 or 13.

15. A computer readable medium having stored thereon the program element of claim 14.
